# EUROPÄISCHE PATENTANMELDUNG

(11) **EP 1 522 851 A1**
(43) Veröffentlichungstag der Anmeldung: **13.04.2005**
(21) Anmeldenummer: 04019953.1
(22) Anmeldetag: 23.08.2004
(51) Int. Cl.: G01N 33/44, B07C 5/34

(54) **Verfahren zur automatischen Analyse von polymerhaltigem Abfall und Analyseautomat**

(30) Priorität: 06.10.2003 DE 10346768
(71) Anmelder: Zimmer AG, 60388 Frankfurt (DE)
(72) Erfinder: Kämpf, Rudolf, Dr., 63584 Haingründau (DE); Reinhard, Wolf, 63517 Rodenbach (DE)
(74) Vertreter: Grünecker, Kinkeldey, Stockmair & Schwanhäusser Anwaltssozietät

(57) **Zusammenfassung**

Die Erfindung betrifft ein Verfahren zur Analyse von polymerhaltigen Abfallgegenständen (2) , insbesondere von Teppichen, in einer Recycling-Anlage (1) sowie einen Analyseautomaten (7). Um die Polymerbestandteile des Abfallgegenstandes (2) zu bestimmen, wird erfindungsgemäß ein Teil des Abfallgegenstandes (2) verdampft und mittels eines Massenspektrometers (18) untersucht. Durch das erfindungsgemäße Verfahren und den erfindungsgemäßen Analyseautomaten lassen sich die Abfallgegenstände (2) für die weitere Verarbeitung (9, 10, 11) genauer und zuverlässiger während des Transports auf einer Fördereinrichtung (6) klassifizieren.

## Beschreibung

Die Erfindung betrifft ein Verfahren, durch das polymerhaltige Abfallgegenstände, wie beispielsweise Teppiche, vor dem Recyceln hinsichtlich der in ihnen enthaltenen Polymere während des Transports analysiert werden. Die Erfindung betrifft ferner einen Analyseautomaten mit einer Sonde, durch den polymerhaltige Abfallgegenstände analysierbar sind.

Das Recycling von Kunststoffen, insbesondere von Polymeren, aus Abfällen der Industrie und der Haushalte nimmt aufgrund gesetzlicher Bestimmungen zum Umweltschutz eine immer stärker werdende Rolle ein. Eine solche gesetzliche Bestimmung liegt beispielsweise dem Pfandflaschen-System der Getränke-Industrie zugrunde, bei dem Flaschen aus Polyethylenterephthalat (PET) recycelt werden. Durch ein kontrolliertes Sammelsystem werden sortenreine und herkunftsbelegte Flaschenabfälle gesammelt und wieder verwertet. Das Pfandflaschen-System ist hinsichtlich der technischen Anforderungen an die Wiederverwertung recht unproblematisch, da lediglich ein einziger Kunststoff, nämlich Polyethylenterephthalat, anfällt und recycelt werden muss. Dies ermöglicht es, gezielte, auf die Eigenschaften von Polyethylenterephthalat abgestimmte Verfahren zu verwenden, beispielsweise um die PET-Flaschen von Verunreinigungen zu befreien. Gemäß der Lehre der EP-A-0 376 119 kann beispielsweise superkritisches Kohlendioxid zur Reinigung der Flaschen aus Polyethylenterephthalat verwendet werden.

Gleiches gilt für andere Bereiche, bei denen Abfallgegenstände aus sortenreinen Kunststoffen recycelt werden. Als Beispiel sei hier das Verfahren der US-A-2002 0128335 genannt, das ausschließlich zur Rückgewinnung von Fluorpolymeren eingesetzt werden kann.

Die üblicherweise in Haushalten und in der Industrie anfallenden Kunststoff-Abfälle weisen jedoch im Unterschied zu den sortenreinen Kunststoffen ein Gemisch aus unterschiedlichen Kunststoffen und Polymeren auf oder sind verschmutzt, so dass die aus diesen Gemischen gewonnenen Recyclate nicht mehr die Eigenschaften der Ausgangsrohstoffe aufweisen. Die bei derartigen Abfallgegenständen zur Wiederverwertung einzusetzenden Vorrichtungen und Verfahren sind wesentlich komplexer als die Vorrichtungen und Verfahren zur Wiederverwertung sortenreiner Kunststoffe.

Eine mengenmäßig große Abfallgruppe aus einem Gemisch unterschiedlicher Polymere stellt beispielsweise Teppichauslegeware dar, deren Wiederaufbereitung bislang noch nicht einer gesetzlichen Regelung unterliegt. Bei der Renovierung öffentlicher und privater Räume fallen nach einer Nutzungsdauer von etwa fünf bis neun Jahren nahezu turnusgemäß größere Mengen gebrauchter Teppichböden und bei neuer Verlegung Verschnittverluste an, die einer Wertstoff-Rückgewinnung zugeführt werden sollten. Eine wirtschaftlich sinnvolle Wiederverwertung setzt jedoch eine Auftrennung der Teppichmaterialien in die einzelnen Komponenten voraus. So besteht der größte Teil der ausgelegten Tufting-Teppiche im Wesentlichen aus Polymerfasern aus Polyamid-6, Polyamid-6,6, Polyethylenterephthalat, Polybutylenterephthalat oder bei preisgünstigeren Typen aus Polypropylen. Der aus diesen Fasern gefertigte Nutzflor ist auf einem Trägermaterial oder Tufting-Grund aus Polypropylen- und/oder Naturfasergewebe aufgebracht. Der Teppichrücken, der mit dem Boden in Berührung kommt, besteht aus einem mit Kreide oder anorganischen Zuschlagsstoffen gefüllten Styrol-Butadien-Latex-Schaum und dient der Trittschalldämmung. Neuerdings werden Zweitrücken nur aus Polypropylen- oder Naturfasergewebe ohne geschäumten Rücken produziert. Die zu entsorgenden Teppichreste können auch erhebliche Menge der unterschiedlichsten Verschmutzungen, wie Erde, Straßenstaub, Teppichkleber, Reinigungsmittel, Lebensmittelreste, etc. enthalten. Mitunter ergeben sich bei einer Sortierung und Sichtung Teppichreste, die sich aufgrund zu starker Verschmutzung als ungeeignet für eine Aufbereitung herausstellen und verbrannt werden.

Aus wirtschaftlichen Gesichtspunkten ist vor allem die erneute Gewinnung von Polymeren aus den Materialien der Abfallgegenstände, die dem Wiederverwertungskreislauf zugeführt werden, durch Spaltung in die Rohstoffe, deren Reinigung und anschließende Polymerisation zur neuen Polymeren interessant. Derartige Wiederverwertungsverfahren sollten es ermöglichen, die Monomere so rein wiederzugewinnen, dass sie in gleicher Weise wie ein frischer Rohstoff wieder zur Polymer-Synthese eingesetzt werden können. Eine Übersicht über thermische Dekompositionsverfahren und Pyrolyseverfahren, mit denen eine solche Wiederverwertung durchgeführt werden kann, findet sich beispielsweise in der EP-A-1 122 293 und in der US-A-5,359,061.

Ein weiteres Verfahren zur Wiederverwertung von Polymeren ist in der US-A-5,872,205 beschrieben, bei dem Abfallstoffe aus einer Polymer-Mischung basierend auf der unterschiedlichen Viskosität der einzelnen Polymere in einer Polymerschmelze getrennt werden. Wie allerdings in dieser Druckschrift selbst ausgeführt ist, ist eine komplette Trennung der Polymere allein aufgrund ihrer unterschiedlichen Schmelze-Viskositäten nicht möglich, es bleiben stets Verunreinigungen getrennten Polymeren zurück.

Eine Reihe von Analyseverfahren beruht darauf, die Reflektions- oder Absorptionseigenschaften der Abfallgegenstände im Infrarot-Bereich zu untersuchen, um den Abfall entsprechend den enthaltenen Polymeren zu trennen.

So ist in der US-A-5,134,291 die Verwendung von Streulicht im nahen Infrarotbereich beschrieben, um Abfallgegenstände in Gruppen ähnlicher Polymere einzuteilen. Eine genaue Einteilung der Polymere zur Erzielung qualitativ hochwertiger Rezyklate ist allerdings mit diesem Verfahren nicht möglich.

Gemäß der Lehre der US-A-5,512,752 wird das Absorptionsspektrum im Nah-Infrarot-Bereich eines unbekannten Kunststoffes gemessen. Zur Bestimmung des Kunststoffes werden das Absorptionsspektrum und die erste und zweite Ableitung des Absorptionsspektrums mit gespeicherten Werten verglichen.

Ähnlich wird bei dem Verfahren der US-A-5,510,619 vorgegangen, wo das Reflektionsspektrum im mittleren Infrarot-Bereich erfasst wird, um Kunststoffe zu identifizieren. Dabei wird von dem erfassten Infrarot-Spektrum die erste Ableitung mit Bezug auf die Wellenzahl gebildet und mit der ersten Ableitung eines Referenz-Infrarot-Spektrums verglichen.

Neben Infrarot-Spektren werden auch Fluoreszenz-Spektren und UV-induzierte Emissionsspektren zur Identifikation von Kunststoffen verwendet, wie in der US-A-5,256,880 beschrieben ist.

In der WO-A-98 019800, die nach Auffassung der Anmelderin den nächstkommenden Stand der Technik bildet, ist ein Sortierverfahren für Kunststoff-Abfälle beschrieben, das auf einer Auswertung des Spektrums der Raman-Streuung beruht. Allerdings ist bei diesem Verfahren, wie im Übrigen auch bei den Infrarot-basierten Verfahren nachteilig, dass beispielsweise Polyamid-6 und Polyamid-6,6 aufgrund ihrer sehr ähnlichen Spektren nicht oder nur mit hohem Aufwand und einer hohen Fehlerquote zu unterscheiden sind. Dies verursacht trotz aufwendiger Analyseverfahren in den nachfolgenden Verarbeitungsstufen, wie beispielsweise einer Spaltung oder Compoundierung einerseits Betriebsstörungen und andererseits Qualitätsverluste bei den Recyclaten.

Der Erfindung liegt daher die Aufgabe zugrunde, ein Verfahren zu schaffen, mit dem die im Abfall enthaltenen Kunststoffe, insbesondere in Teppichen und Auslegeware, in einer Recycling-Anlage zuverlässig und genau identifiziert werden können, um sie sortieren zu können und Recyclate hoher Qualität zu erhalten.

Diese Aufgabe wird für das eingangs genannte Verfahren erfindeangsgemäß dadurch gelöst, dass ein Teil des Abfallgegenstandes in den gasförmigen Zustand überführt und das so erhaltene Gas mittels eines Massenspektrometers massenspektroskopisch untersucht wird.

Diese Lösung ist einfach und vermeidet die mit der optischen Analyse von Reflektionsund Absorptionsspektren bzw. von Fluoreszenz- und Raman-Spektren verbundenen Probleme, da durch die massenspektroskopische Untersuchung die Polymerbestandteile des Abfallgegenstandes mit wenig Aufwand und hoher Zuverlässigkeit und in Echtzeit analysiert werden können. Die Abfallgegenstände können dann in Abhängigkeit von der Analyse sortiert werden.

Die Funktionsweise der Massenspektroskopie ist beispielsweise in: D.A.Skoog und J.J. Leary, "Instrumentelle Analytik", Springer-Verlag, Berlin, Heidelberg, New York, 1996, beschrieben. Diese Referenz wird hiermit ausdrücklich in den Offenbarungsgehalt aufgenommen.

Die Verwendung der Massenspektroskopie im Rahmen der erfindungsgemäßen Lösung unterscheidet sich wesentlich vom üblichen Einsatzgebiet der Massenspektroskopie, wie es beispielsweise aus der EP-A-1 122 993 und der US-A-5,359,061 bekannt ist. Dort dient die massenspektroskopische Untersuchung lediglich dazu, das Ergebnis der Pyrolyse stichpunktartig zu überprüfen. Im Gegensatz zu dieser Verwendung wird erfindungsgemäß die Massenspektroskopie zur laufenden Überwachung und Klassifizierung des einer Recycling-Anlage zugeführten Abfalls vor dessen Wiederverwertung eingesetzt, so dass eine zuverlässige und genaue Sortierung der Abfälle nach den enthaltenen Polymeren stattfinden kann.

Die oben genannte Aufgabe wird auch durch einen Analyseautomaten gelöst, der in einer Anlage zum Recyceln von polymerhaltigen Abfallgegenständen, wie beispielsweise Teppichen, einbaubar ausgestaltet ist, so dass bestehende Anlagen nachgerüstet werden können. Der Analyseautomat weist eine Sonde mit einer Verdampfungseinrichtung und einer Gasleitung auf, wobei durch das Verdampfungsmodul ein Teil des zu recycelnden Abfallgegenstandes zu einem Gas verdampfbar ist, und wobei die Gasleitung an ein Massenspektrometer anschließbar ausgestaltet ist und durch die Gasleitung das Gas dem Massenspektrometer zur Erfassung eines Massenspektrums zuführbar ist Der erfindungsgemäße Analyseautomat weist ferner eine mit dem Massenspektrometer datenübertragend verbindbare Auswerteeinheit auf, durch die in Abhängigkeit vom erfassten Massenspektrum ein für die Zusammensetzung des Polymers repräsentatives Signal ausgebbar ist.

Der Vorteil der Massenspektroskopie bei der Analyse der Abfallgegenstände besteht insbesondere darin, dass das vom Massenspektrometer gemessene Massenspektrum eines Kunststoffgemisches lediglich eine Addition der einzelnen Massenspektren der Bestandteile dieser Kunststoffe ist. Somit lassen sich durch einfache Lösung linearer Gleichungssysteme mit geringem Rechenaufwand die Zusammensetzungen der durch die Recycling-Anlage transportierten und von Massenspektrometer analysierten Abfallgegenstände bestimmen. Das Auswerteverfahren kann somit schnell genug durchgeführt werden, so dass noch während des Transports der Abfallgegenstände durch die Anlage die Bestandteile bestimmt und sie entsprechenden, an ihre Bestandteile angepassten Verarbeitungsschritten zugeführt werden können. Somit erlauben das erfindungsgemäße Verfahren und der erfindungsgemäße Analyseautomat eine fortlaufende Analyse der durch die Recycling-Anlage transportieren Abfallgegenstände in einem kontinuierlichen Modus während des Transports der Abfallgegenstände.

Das Massenspektrometer kann insbesondere auf bestimmte Arten von Abfallgegenständen geeicht werden, wie beispielsweise Teppiche, indem Massenspektren der einzelnen Bestandteile bzw. Fragmente der Abfallgegenstände in einem Speicher der Auswerteeinheit als Eichspektren hinterlegt werden. In Abhängigkeit vom Vergleich des gemessenen Spektrums mit den gespeicherten Eichspektren lässt sich dann die Zusammensetzung des Abfallgegenstandes analysieren.

Im Folgenden werden verschiedene vorteilhafte Weiterbildungen des erfindungsgemäßen Verfahrens und des erfindungsgemäßen Analyseautomats beschrieben. Die unterschiedlichen Ausgestaltungen können unabhängig voneinander in beliebiger Kombination verwendet werden.

So kann beispielsweise die Geschwindigkeit des Auswertevorganges dadurch erhöht werden, dass lediglich die Maxima bzw. Spitzen der gemessenen Massenspektren mit den Maxima bzw. Spitzen der Eichspektren verglichen werden. Durch diese Maßnahme wird der Vergleich auf wenige Datenpunkte reduziert, so dass nur ein geringer Rechenaufwand zur Identifizierung der in einem Abfallgegenstand enthaltenen Polymere betrieben werden muss.

Da die Zusammensetzung der in einer Recycling-Anlage anfallenden Kunststoffe eine große Bandbreite aufweisen kann, ist es gemäß einer Ausgestaltung von Vorteil, wenn im Zuge der massenspektroskopischen Untersuchung Massenspektren über einen Bereich von 1 amu (atomic mass unit) bis 5000 amu, vorzugsweise 1 amu bis 2000 amu bzw. 1 amu bis 1000 amu erfasst werden.

Eine weitere Reihe von vorteilhaften Ausgestaltungen befasst sich mit der Gasleitung, mit der das verdampfte Gas des zu analysierenden Abfallgegenstandes zum Massenspektrometer geleitet wird. So kann diese Gasleitung beispielsweise vorteilhaft beheizt sein.

Insbesondere kann die Temperatur der Gasleitung mindestens 10°C oberhalb des Schmelzpunktes des Polymers oder der am höchsten schmelzenden Komponente liegen, so dass in der Gasleitung sich keine Feststoffe ablagern können. Die Gefahr von Ablagerungen kann gemäß einer vorteilhaften Weiterbildung auch dadurch verringert werden, dass die Temperatur der Gasleitung mindestens 70°C oberhalb des Schmelzpunktes des Polymers oder der am höchsten schmelzenden Komponente liegt.

Falls vermieden werden soll, dass sich in der Gasleitung zum Massenspektrometer Kondensate niederschlagen, so kann die Temperatur der Leitung oberhalb der Kondensationstemperatur des zum Massenspektrometer geleiteten Gases liegen. Diese Temperatur kann beispielsweise bei mindestens 250°C liegen.

In der Gasleitung zum Massenspektrometer kann gemäß einer weiteren Ausgestaltung ein Verdünnungsgasstrom aus Stickstoff oder Helium dem Gasstrom zugesetzt werden, um die Konzentration des Gases herabzusetzen.

Damit nachfolgende Messungen nicht beeinflusst werden, kann gemäß einer weiteren Reihe von vorteilhaften Ausgestaltungen eine Spüleinrichtung- vorgesehen sein, durch die die Gasleitung nach einer Probenentnahme gereinigt wird. Damit kann sichergestellt werden, dass die Messungen nicht durch Ablagerungen oder Verunreinigungen aus vorangegangenen Probenentnahmen beeinflusst werden.

Eine solche Reinigung kann beispielsweise durch Ausheizen der Leitung geschehen, so dass Rückstände an der Leitung und im Inneren der Leitung verbrannt werden und die nachfolgende Messung nicht mehr beeinflussen können. Alternativ oder zusätzlich zur Reinigung mittels Ausheizen kann auch ein Spülgas durch die Leitung geleitet werden, vorzugsweise ein Inertgas wie Helium oder Stickstoff. Das Spülgas kann ebenfalls eine hohe Temperatur aufweisen, durch die Rückstände in der Leitung verbrannt werden. Insbesondere kann die Temperatur des Spülgases wie oben bei der Temperatur der Gasleitung festgelegt werden, also beispielsweise ebenfalls oberhalb der Kondensationsgrenze des Gases liegen.

Damit das Verfahren während des Transports der Abfallgegenstände an der Sonde bzw. dem Analyseautomaten vorbei trotz der Reinigung der Leitung unterbrechungsfrei durchgeführt werden kann, sind gemäß einer weiteren vorteilhaften Ausgestaltung mindestens zwei Sonden vorgesehen, die abwechselnd eine Gasprobe von den an dem Analyseautomaten vorbeitransportierten Abfallgegenständen entnehmen. Insbesondere kann bei dieser Ausgestaltung die eine Sonde automatisch gereinigt werden, während die andere Sonde die Messung vornimmt.

Eine Kalibrierung des Analyseautomaten kann vorzugsweise dadurch erfolgen, dass vorab die Massenspektren aller bekannten und in den Abfallgegenständen, insbesondere den Teppichen, vorkommenden Polymere und der während der Verdampfung auftretenden Abbauprodukte mehrfach mit verschiedenen Aufheizraten der Verdampfungseinrichtung gemessen und als Eichspektren im Analyseautomaten abgelegt werden. Wird dann im Betrieb der Recyclinganlage das Massenspektrum eines Abfallgegenstandes gemessen, so wird dieses mit den abgespeicherten Eichspektren verglichen und dasjenige Eichspektrum ausgewählt, dessen Abweichung vom gemessenen Massenspektrum am kleinsten ist. Die Abweichung kann beispielsweise in Form der Summe der Fehlerquadrate über das Massenspektrum berechnet werden.

Da das ausgewählte Eichspektrum für eine bestimmte Zusammensetzung des Polymers repräsentativ ist, kann die Zusammensetzung des Abfallgegenstandes so bestimmt werden, dass der Abfallgegenstand in Abhängigkeit vom Ergebnis der massenspektroskopischen Untersuchung sortiert wird. In einer alternativen oder zusätzlichen Vorgehensweise werden die Eichspektren in Form von Fragmentierungsspektren bestimmt, wobei ein Fragmentierungsspektrum den Bestandteilen eines bestimmten Polymers entspricht. Bei dieser Vorgehensweise wird nach den Eich- bzw. Fragmentierungsspektren gesucht, durch deren Addition das gemessene Massenspektrum erhalten wird. Die Fragmentierungsspektren der einzelnen Polymere sind dabei entsprechend des Anteils des Polymers im Abfallgegenstand entsprechend im Massenspektrum gewichtet. Im Gegensatz zur vorangegangenen Vorgehensweise wird bei dieser Vorgehensweise die Zusammensetzung des Abfallgegenstandes analysiert. In Abhängigkeit von dieser Analyse kann dann der Abfallgegenstand klassifiziert werden.

Im Folgenden wird die Erfindung anhand von unterschiedlichen Ausführungsformen mit Bezug auf die Zeichnungen beispielhaft genauer erläutert. Dabei werden bei den unterschiedlichen Ausführungsformen für Merkmale gleichen Aufbaus oder gleicher Funktion die gleichen Bezugszeichen verwendet.

Wie ferner aus der vorangegangenen Beschreibung der mit den einzelnen Merkmalen verbundenen Vorteile hervorgeht, können die Merkmale der unterschiedlichen Ausführungsformen beliebig kombiniert werden und es können einzelne Merkmale der Ausführungsformen auch zur Gänze weggelassen werden.

Es zeigen:
- Fig. 1: eine erste Ausführungsform einer Abfallrecycling-Anlage mit einem erfindungsgemäßen Analyseautomaten in einer schematischen Darstellung;
- Fig. 2: eine zweite Ausführungsform einer Abfallrecycling-Anlage, insbesondere für Teppiche, in einer schematischen Darstellung;
- Fig. 3: eine erste Ausführungsform einer Sonde eines erfindungsgemäßen Analyseautomaten in einer schematischen Darstellung;
- Fig. 4: eine weitere Ausführungsform einer Sonde eines erfindungsgemäßen Analyseautomaten in einer schematischen Darstellung;
- Fig. 5: eine weitere Ausführungsform einer Sonde eines erfindungsgemäßen Analyseautomaten in einer schematischen Darstellung;
- Fig. 6: ein Leitungssystem eines erfindungsgemäßen Analyseautomaten in einer schematischen Darstellung;
- Fig. 7: eine schematische Darstellung eines Massenspektrums zur Analyse der Zusammensetzung eines Abfallgegenstandes.

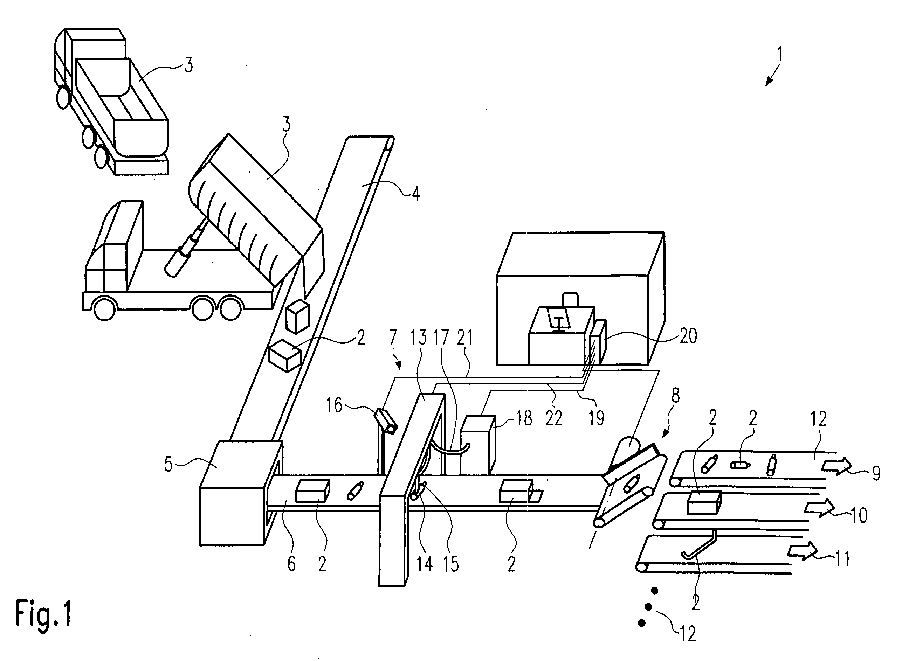

Fig. 1 zeigt schematisch einen Ausschnitt aus einer Anlage 1 zum Recyceln von polymerhaltigen Kunststoffabfällen 2, die, beispielsweise per Lastwagen 3 von einer Sammelstelle vorsortiert, d. h. befreit von nicht-kunststoffhaltigem, Abfall herbeigeschafft werden. Die Kunststoffabfälle 2 werden von einem Förderband von dem Lastwagen 3 zu einer Vorrichtung 5 gefördert, durch welche die Abfallgegenstände 2 auf einem weiteren Förderband 6 gesammelt und ausgerichtet werden. Das Förderband 6 ist mit einem erfindungsgemäßen Analyseautomaten 7 versehen, der die Zusammensetzung der Abfallgegenstände 2 bestimmt und in Abhängigkeit von deren Zusammensetzung eine Sortiereinrichtung 8, beispielsweise in Form einer Weiche bedient. Durch die Sortiereinrichtung 8 werden die Abfallgegenstände 2 in Abhängigkeit von ihrer Zusammensetzung und von den in ihnen enthaltenen Polymeren unterschiedlichen Wiedergewinnungs- und Verarbeitungsverfahren zugeführt, wie dies durch die Pfeile 9, 10, 11 angedeutet ist.

Beispielsweise können durch den Analyseautomaten 7 Flaschen aus Polyethylenterephthalat erkannt und auf einem entsprechenden Förderweg 12 einer auf Polyethylentherephthalat-Flaschen zugeschnittenen Weiterverarbeitung zugeleitet werden.

Ebenso können beispielsweise stark verschmutzte Gegenstände durch den Analysenautomaten 7 erkannt und durch Steuerung der Sortiereinrichtung 8 einer Verbrennung 10 zugeführt werden, da sie aufgrund ihrer starken Verschmutzung, beispielsweise durch toxische Stoffe, nicht mehr zur Wiederaufbereitung geeignet sind.

Ebenso können für Abfallgegenstände 2, die andere Kunststoffe als Polyethylenterephtalat, wie beispielsweise Polyurethan, Polyamid, Polyester, Polyvinylchlorid, um nur einige zu nennen, enthalten, entsprechende Wiederaufbereitungsverfahren 11 und, durch Punkte angedeutet, 12 vorgesehen sein.

Der Analyseautomat 7 besteht bei der in Fig. 1 dargestellten Ausführungsform aus einer in Form eines Portalroboters 13 ausgebildeten Positioniereinrichtung, durch die eine Sonde 14 in Form einer Gasleitung nahe an oder in Kontakt mit einem zu untersuchenden Abfallgegenstand 15 gebracht werden kann. Der Aufbau der Sonde 14 ist weiter unten genauer beschrieben. Die Positioniereinrichtung 13 ist derart beweglich ausgestaltet, dass die Sonde 14 beliebige Gegenstände auf dem Förderband 6 abtasten kann. Beispielsweise weist die Positioniereinrichtung drei translatorisch angetriebene, im Wesentlichen zueinander senkrechte, Freiheitsgrade oder Achsen auf sowie mindestens eine rotatorisch angetriebene Achse.

Über eine Überwachungsvorrichtung 16, beispielsweise eine Videokamera mit nachgeschalteter Bilddatenverarbeitung, wird die Lage des zu untersuchenden Gegenstandes auf dem Förderband 6 erfasst und die Sonde 14 während des Transports des Abfallgegenstandes 2 auf der Fördereinrichtung 6 zielgenau an den zu untersuchenden Abfallgegenstand 2 herangefahren.

Die Sonde 14 entnimmt eine Probe vom Abfallgegenstand 15, indem ein kleiner Teil des gerade analysierten Abfallgegenstandes 15 verdampft wird. Dieser Dampf wird über eine Leitung 17 einem Massenspektrometer 18, beispielsweise einem Quadropol-Breitbandmassenspektrometer der Firma Balzers vom Typ HPA 2000 mit einem Messbereich von 1 amu bis 1.000 amu, zugeführt.

Im Massenspektrometer werden die Massenspektren der Bestandteile bzw. Fragmente des Gases erfasst und in Signalform über eine Datenleitung 19 einer Auswerte- und Steuereinheit 20, beispielsweise in Form eines Computers zugeführt. In dem PC werden die gemessenen Massenspektren mit vorab bestimmten, gespeicherten Eichspektren verglichen. Die Eichspektren wurden beispielsweise vorab aus der Analyse von Gegenständen bekannter Zusammensetzung erhalten. Bei einer Übereinstimung eines gemessehen Massenspektrums mit einem gespeicherten Eichspektrum wird die Sortiereinrichtung 8 durch die Steuereinheit 20 entsprechend der dem Eichspektrum zugeordneten Zusammensetzung so angesteuert, dass der Abfallgegenstand umweltgerecht entsorgt werden kann.

Weist der gerade analysierte Abfallgegenstand 15 mehrere Polymere als Bestandteile auf, so ergibt sich sein Massenspektrum als Summe der Eichspektren dieser Bestandteile, wobei die Anteile der Eichspektren an der Summe entsprechend dem Anteil des Bestandteiles im Abfallgegenstand gewichtet sind. Auf diese Weise kann die Sortiereinrichtung 8 auch in Abhängigkeit von den bestimmten Bestandteilen und ihren Anteilen zugesteuert werden.

Die Steuereinheit 20 kann zusätzlich noch den Portalroboter 13 in Abhängigkeit von den Signalen der Überwachungsvorrichtung 16 steuern, wie durch die insbesondere bidirektionalen Datenleitungen 21, 22 angedeutet ist.

Bei der Synchronisierung der Steuerung der Sortiereinrichtung 8 mit der Bewegung der Abfallgegenstände 2 auf der Fördereinrichtung 6 kann der Abstand des Analyseautomaten 7 von der Sortiereinrichtung 8 und die Geschwindigkeit des Förderbandes 6 oder einer anderen, gleichwertigen Fördereinrichtung berücksichtigt werden, so dass die Sortiereinrichtung betätigt wird, wenn der entsprechende Abfallgegenstand vorbeitransportiert wird.

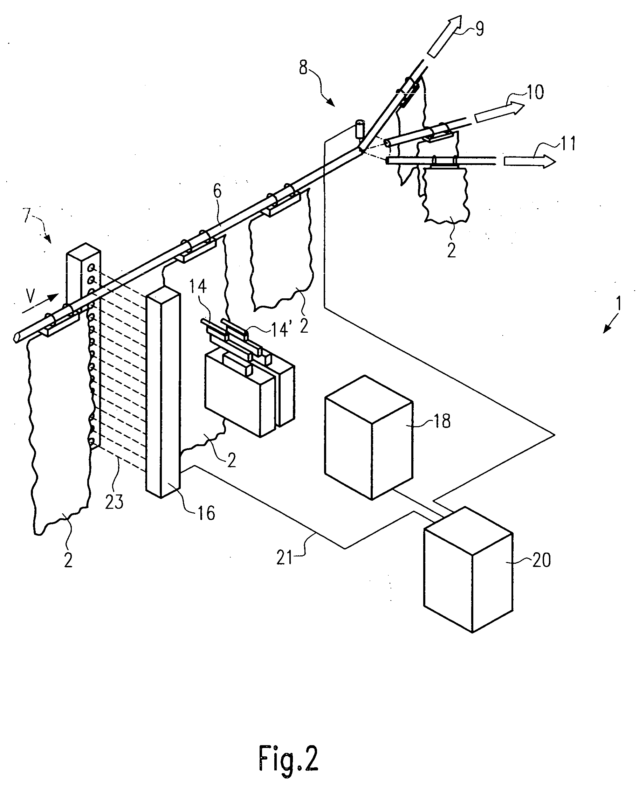

Fig. 2 zeigt eine schematische Darstellung einer weiteren Anlage 1 zur Sortierung von polymerhaltigen Abfallgegenständen 2, wobei die Anlage 1 der Fig. 2 speziell zur Analyse und zum Sortieren von Teppichen ausgestaltet ist.

Die Teppiche 2 sind auf einer als Hängeförderer ausgebildeten Fördereinrichtung 6 eingehängt und passieren die Überwachungsvorrichtung 16, die bei der Ausführungsform der Fig. 2 als ein Feld übereinander angeordneter Lichtschranken 23 ausgestaltet ist.

Die Teppiche 2 passieren die Überwachungseinrichtung 16 mit einer vorbestimmten Geschwindigkeit v der Fördereinrichtung 6 und decken bei ihrer Passage, abhängig von ihrer Größe und Form, einen Teil der Lichtschranken 23 ab. Über die Datenleitung 21 kann die Auswerteeinheit 20 anhand des Signals von der Überwachungseinrichtung 16 und anhand der Fördergeschwindigkeit v die Form der Teppiche 2 bestimmen und eine Sonde 14 so zustellen, dass sie eine Probe der Teppichoberfläche entnehmen kann. Eine weitere, identische Sonde (nicht gezeigt) kann von der anderen Seite der Teppichoberfläche zugestellt werden, um sowohl Vorder- als auch Rückseite gleichzeitig analysieren zu können.

Wie in Fig. 2 dargestellt ist, können auf jeweils einer Seite eines Teppichs 2 zwei Sonden 14, 14' angeordnet sein, die abwechselnd an den Abfallgegenständen 2 Analysen vornehmen. Auf diese Weise kann eine Sonde, im Bild der Fig. 2 ist dies die Sonde 14', gereinigt werden, während die andere Sonde 14 die Probe entnimmt.

Die Probe wird in Gasform dem Massenspektrometer 18 zugeführt, die Steuer- und Auswerteeinheit 20 vergleicht die gemessenen Massenspektren mit den gespeicherten Eichspektren und betätigt in Abhängigkeit von diesem Vergleich die Sortiereinrichtung 8, um die Teppiche unterschiedlichen Verarbeitungsprozessen 9, 10, 11 zuzuführen.

Natürlich können die Steuereinheit und die Auswerteeinheit 20 auch in Form getrennter Geräte ausgeführt sein, die lediglich durch Datenleitungen verbunden sind.

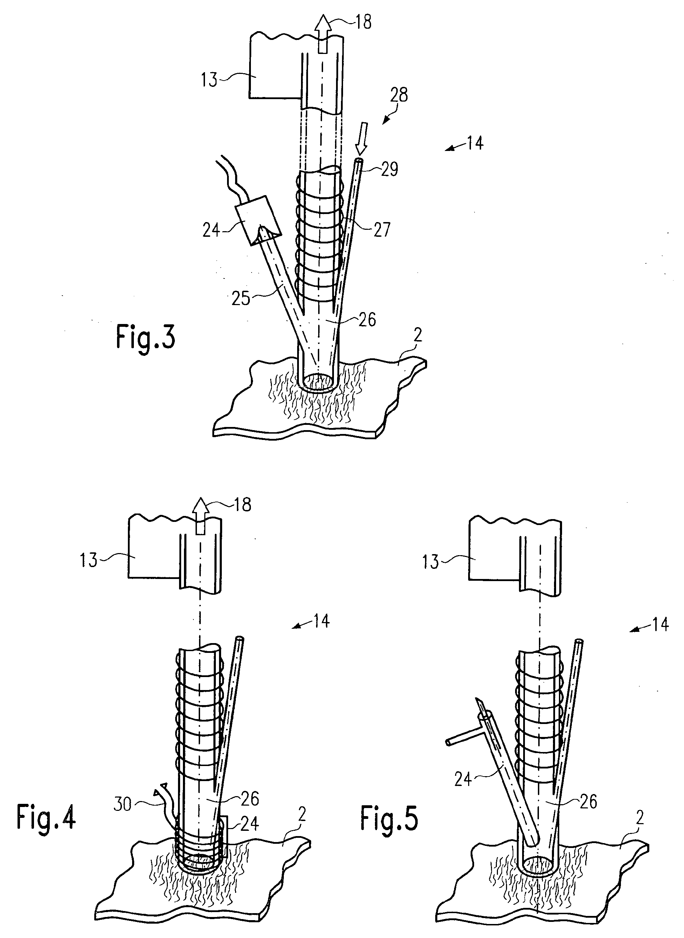

Fig. 3 zeigt in einem schematischen Detail eine erste Ausführungsform der Sonde 14 des Analyseautomaten 7.

Die Sonde 14 weist eine Verdämpfungseinrichtung 24 in Form eines Lasers auf, der einen Laserstrahl 25 auf den zu untersuchenden Abfallgegenstand 2 richtet. Durch den Laserstrahl wird ein Teil des Abfallgegenstandes 2 verdampft. Der Dampf wird durch ein vorzugsweise kapillarförmiges Sondenrohr 26 abgesaugt und zum Massenspektrometer 18 (in Fig. 3 nicht dargestellt) geleitet.

Mittels einer Sondenheizung 27 wird das Sondenrohr 14 auf eine Temperatur oberhalb des Kondensationspunktes des von der Verdampfungseinrichtung 24 erzeugten Dampfes aufgeheizt. Diese Temperatur kann 10°C bzw. vorzugsweise 70°C oberhalb der Schmelztemperatur des Kunststoffes des Abfallgegenstandes 2 oder der Komponente des Abfallgegenstandes 2 mit der höchsten Schmelztemperatur liegen. Insbesondere kann die Heiztemperatur, auf die das Sondenrohr 26 aufgeheizt wird, oberhalb von 250°C liegen und vorzugsweise um die 270°C betragen.

Die Sonde 14 ist, wie in Fig. 3 lediglich schematisch dargestellt, in einer Positioniereinrichtung 13 gehalten, die beispielsweise in Form eines pneumatisch oder elektrisch angetriebenen Mehrachs-Roboters bzw. einer Zustelleinrichtung ausgestaltet ist.

Durch die Positioniereinrichtung ist sichergestellt, dass jeder Abfallgegenstand 2 auf der Fördereinrichtung 6 von der Sonde erreicht werden kann.

Des Weiteren weist die Sonde 14 eine Spül- bzw. Reinigungseinrichtung 28 auf, mit der die Sonde 14 gereinigt bzw. gespült wird, wenn keine Probe von Abfallgegenständen 2 entnommen wird.

Die Spül- bzw. Reinigungseinrichtung 28 weist eine Zuleitung 29 auf, durch die ein Inertgas in das Sondenrohr 27 eingeleitet werden kann, so dass das Sondenrohr durchspült und von Kondensationsresten des verdampften Abfallgegenstandes 2 befreit wird. Vorzugsweise wird während des Spülvorgangs die Sonde 14 aus ihrer Verbindung mit dem Massenspektrometer 18 gelöst. Um das Spülgas nicht an die Umgebung abzugeben, kann die Sonde 14 während des Spülvorgangs mit einer in Fig. 2 nicht dargestellten Gasableitung verbunden sein, durch die das Spülgas gereinigt und/oder aufgefangen wird.

Zusätzlich kann die Sondenheizung 27 als Teil der Reinigungseinrichtung betrieben werden, indem sie das Sonden- bzw. Kapillarrohr 27 auf eine Temperatur aufheizt, in der Rückstände an der Wand des Kapillar- bzw. Sondenrohres und im Inneren des Sondenrohres verbrannt werden. Um auch bei einer Vielzahl von Messungen eine hohe Messgenauigkeit beizubehalten, ist es von Vorteil, wenn während der gesamten Zeit, in der die Sonde 14 keine Probe entnimmt, eine Spülung mit dem Spülgas stattfindet.

Das Spülgas kann ebenfalls auf eine Temperatur aufgeheizt sein, die zu einer Verbrennung der Rückstände im Kapillarrohr führt, die Temperatur des Inertgases liegt vorzugsweise oberhalb der Kondensationstemperatur des von der Verdampfungseinrichtung 24 erzeugten Dampfes.

In den Fig. 4 und 5 sind zwei weitere Ausführungsformen der Sonde 14 beschrieben; die als alternative oder ergänzende Ausgestaltungen zu der Ausführungsform der Fig. 3 verwendet werden können. Der Einfachheit halber wird im Folgenden lediglich auf die Unterschiede zur Ausführungsform der Fig. 3 eingegangen.

In Fig. 4 ist die Verdampfungseinrichtung 24 in Form einer elektrischen Heizvorrichtung ausgestaltet, die in einem Bereich der Sonde 14 angeordnet ist, der mit dem Abfallgegenstand 2 in Berührung kommt, vorzugsweise also an der Mündung des Kapillarrohres 26. Über eine lediglich schematisch dargestellte Regelungseinrichtung 30 wird die Temperatur der Verdampfungseinrichtung 24 konstant gehalten.

Die Ausführungsform der Fig. 4 hat den Vorteil, dass durch die Verdampfungseinrichtung 24 an der Mündung in einer Doppelfunktion gleichzeitig dieser Bereich gereinigt werden kann, indem die Verdampfungseinrichtung 24, wenn keine Proben entnommen werden, auf eine Ausheiztemperatur hochgefahren wird, in der Rückstände im Mündungsbereich des Kapillarrohres 26 verbrennen. Insbesondere der Mündungsbereich ist nämlich überraschend anfällig für Ablagerungen des verdampften Abfallgegenstandes 2, welche nachfolgende Messungen beeinträchtigen können.

Bei der Ausführungsform der Fig. 5 wird ein Gasbrenner als Verdampfungseinrichtung 24 verwendet, dem über eine Zuleitung 31 Brenngas zugeführt wird. Diese Ausführung ist kostengünstig, hat jedoch den Nachteil, dass durch Reaktionen mit dem Brenngas und durch das Brenngas selber die Messungen schwieriger auszuwerten sind.

Bei allen Ausführungsformen kann das Kapillarrohr 26 aus korrosionsbeständigen Stahl mit einer polierten Innenseite oder aus Glas gefertigt sein.

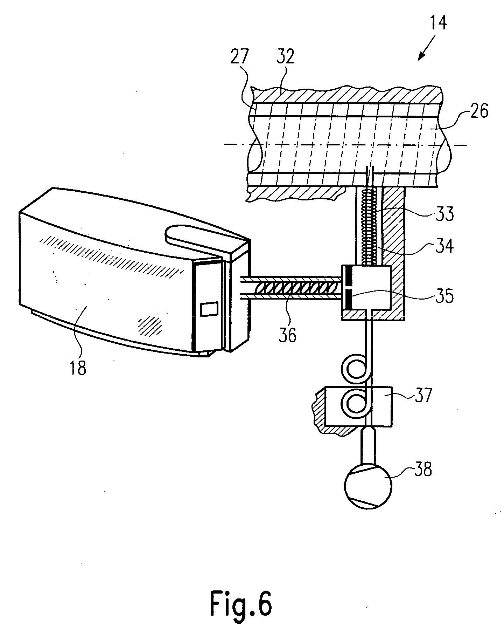

In Fig. 6 ist eine Ausführungsform der Verbindung zwischen der Sonde 14 und dem Massenspektrometer 18 schematisch dargestellt.

Wie in Fig. 6 zu erkennen ist, kann das Kapillarrohr 26 mit der Heizvorrichtung 27 von einer Wärmeisolierung 32 umgeben sein, damit die Wandtemperatur möglichst konstant gehalten wird. Zusätzlich kann dem Dampf von Abfallgegenstand 2 (in Fig. 6 nicht dargestellt) beispielsweise durch die Spülgaszuleitung 29 ein Inertgas wie Heliumgas oder Stickstoff zugeführt werden, um die Gasprobe zu verdünnen.

Aus dem Kapillarrohr 26 wird über eine Ansaugkapillare 33 der Probendampf abgesaugt. Die Absaugkapillare 33 ist mit einer weiteren Heizvorrichtung 34 versehen und im Wesentlichen auf dieselbe Temperatur wie die Sondenleitung 26 aufgeheizt. Über eine Messblende 35, die einen vorbestimmten Volumenstrom sicherstellt und die zur Vermeidung von Kondensationen des Probengases ebenfalls beheizt ist, wird das Probengas durch eine Zuleitungskapillare 36 zum Massenspektrometer 18 geleitet, wo das Massenspektrum der Gasprobe ermittelt wird.

Über eine Kühlfalle 37 wird das Probengas von einer mehrstufigen Vakuumsaugpumpe 38 oder einer lonenpumpe abgeleitet und entsorgt, beispielsweise verbrannt.

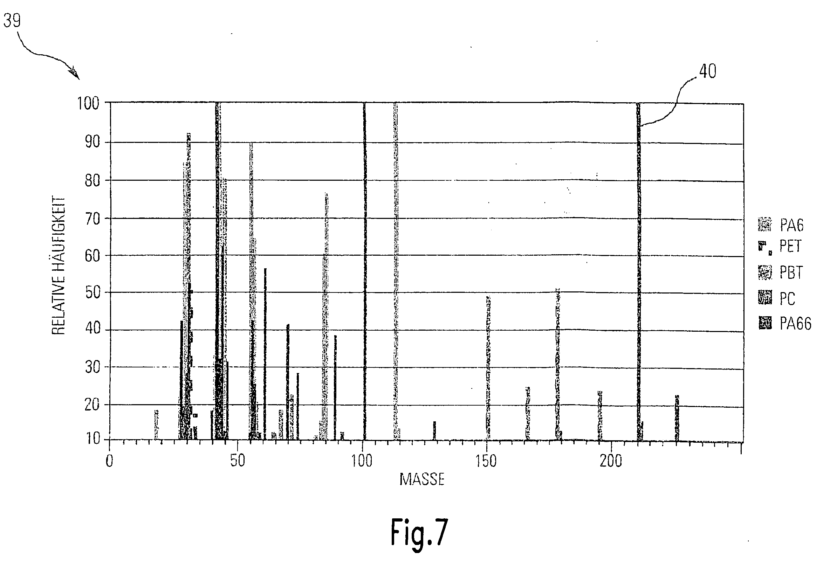

Fig. 7 zeigt ein typisches Massenspektrum 40 eines Teppichrestes. Das Massenspektrum wird dabei auch als Fragmentierungsspektrum bezeichnet, da im Massenspektrometer die Polymere in Bestandteile, die sogenannten Fragmente aufgeknackt werden. Durch das Massenspektrometer wird das Verhältnis der Masse zur Ladung der ionisierten Fragmente ermittelt. Jedes Polymer zeichnet sich dabei durch eine bestimmte Kombination und durch eine bestimmte relative Häufigkeit der Fragmente aus. Somit eignen sich die Fragmentierungsspektren besonders als Eichspektren.

In dem in Fig. 7 schematisch dargestellten Beispiel enthält der Teppichrest die Polymere Polyamid-6 (PA 6), Polyethylenterephthalat (PET), Polybutylenterephthalat (PBT), Polycarbonat (PC) und Polyamid-6,6 (PA 6,6). Wie in Fig. 7 zu erkennen ist, weisen diese Polymere teilweise die gleichen Fragmente, teilweise unterschiedliche Fragmente auf, was an der Lage der Peaks an der mit "Masse" bezeichneten x-Achse zu erkennen ist: Liegen Peaks an der gleichen Stelle, so weisen die Polymere die gleichen Bestandteile auf. Da sich allerdings der Anteil der einzelnen Fragmente bei den Polymeren unterscheidet, ist die Höhe der Peaks, also die relative Häufigkeit dieser Fragmente in den Polymeren, unterschiedlich.

Da für jedes Polymer durch Eichspektren vorab die Zusammensetzung ermittelt wird und sich ein Massenspektrum eines beliebigen Abfallgegenstandes 2, wie es in Fig. 7 gezeigt ist, durch eine lineare Kombination, d.h. eine Addition der einzelnen Fragmentierungsspektren der reinen Polymere in Abhängigkeit von ihrer Häufigkeit im Abfallgegenstand 2, zusammensetzt, kann durch die Massenspektroskopie die Zusammensetzung des gesamten Abfallgegenstandes 2 durch Lösung eines linearen Gleichungssystems auf einfache Weise bestimmt werden.

Dieses Verfahren ist so schnell und so zuverlässig, dass erfindungsgemäß das Verfahren zur Online-Analyse von Abfallgegenständen in Recycling-Anlagen verwendet werden kann.

## Patentansprüche

1. Verfahren, durch das polymerhaltige Abfallgegenstände vor dem Recyceln hinsichtlich der in ihnen enthaltenen Polymere während des Transports analysiert werden, **dadurch gekennzeichnet, dass** ein Teil des Abfallgegenstandes (2) in den gasförmigen Zustand überführt und mittels eines Massenspektrometers (18) massenspektroskopisch untersucht wird.

2. Verfahren nach Anspruch 1, **dadurch gekennzeichnet, dass** ein im Zuge der massenspektroskopischen Untersuchung automatisch erfasstes Massenspektrum mit abgespeicherten, für Polymere repräsentative Eichspektren automatisch verglichen wird und dass der Abfallgegenstand (2) in Abhängigkeit von diesem Vergleich sortiert wird.

3. Verfahren nach Anspruch 2, **dadurch gekennzeichnet, dass** die Maxima (40) und ihre Lage im gemessenen Massenspektrum (39) mit dem Maxima der Eichspektren verglichen werden.

4. Verfahren nach einem der obengenannten Ansprüche, **dadurch gekennzeichnet, dass** die Massenspektren über einen Bereich von 1 amu bis 2000 amu erfasst werden.

5. Verfahren nach einem der obengenannten Ansprüche, **dadurch gekennzeichnet, dass** das Gas durch eine Gasleitung (26) in das Massenspektrometer (18) gesaugt wird.

6. Verfahren nach Anspruch 5, **dadurch gekennzeichnet, dass** die Gasleitung (26) beheizt wird.

7. Verfahren nach Anspruch 5 oder 6, **dadurch gekennzeichnet, dass** die Leitung (26) auf eine Temperatur von mindestens 10°C oberhalb des Schmelzpunktes des Polymers oder der am höchsten schmelzenden Komponente des Abfallgegenstandes (2) liegt.

8. Verfahren nach Anspruch 7, **dadurch gekennzeichnet, dass** die Leitung (26) auf eine Temperatur von mindestens 70°C oberhalb des Schmelzpunktes des Polymers oder der am höchsten schmelzenden Komponente des Abfallgegenstandes geheizt wird.

9. Verfahren nach einem der Ansprüche 6 bis 9, **dadurch gekennzeichnet, dass** die Temperatur der Leitung (26) auf mindestens 250°C geregelt wird.

10. Verfahren nach einem der oben genannten Ansprüche, **dadurch gekennzeichnet, dass** der zum Massenspektrometer (18) geleitete Gasstrom durch ein Inertgas verdünnt wird.

11. Verfahren nach einem der oben genannten Ansprüche, **dadurch gekennzeichnet, dass** die Gasleitung (26) nach einer Probenentnahme automatisch gereinigt wird.

12. Verfahren nach Anspruch 12, **dadurch gekennzeichnet, dass** die Leitung (26) zur Reinigung mit einem Inertgas gespült wird.

13. Verfahren nach einem der Ansprüche 10 bis 12, **dadurch gekennzeichnet, dass** das Inertgas aufgeheizt wird.

14. Verfahren nach einem der oben genannten Ansprüche, **dadurch gekennzeichnet, dass** während des Spülens der Gasleitung (26) eine massenspektroskopische Untersuchung mit einer weiteren Gasleitung (26) durchgeführt wird.

15. Verfahren nach einem der oben genannten Ansprüche, **dadurch gekennzeichnet, dass** die Abfallgegenstände (2) während des Transports durch eine Fördereinrichtung (6) der Anlage (1) analysiert werden.

16. Verfahren nach einem der oben genannten Ansprüche, **dadurch gekennzeichnet, dass** der Abfallgegenstand in Abhängigkeit vom Ergebnis der massenspektroskopischen Untersuchung sortiert wird.

17. Analyseautomat (7), der in eine Anlage (1) zum Recyceln von polymerhaltigen Abfallgegenständen (2) einbaubar ausgestaltet ist, mit einer Sonde (14), die eine Verdampfungseinrichtung (24) und eine Gasleitung (26) umfasst, wobei durch die Verdampfungseinrichtung ein Teil des Abfallgegenstandes zu einem Gas verdampfbar ist, und wobei die Gasleitung (26) an ein Massenspektrometer (18) anschließbar ausgestaltet ist und durch die Gasleitung das Gas dem Massenspektrometer zur Erfassung eines Massenspektrums (39) zuführbar ist, mit einer Auswerteeinheit (20), die datenübertragend mit dem Massenspektrometer (18) verbindbar ausgestaltet ist und durch die in Abhängigkeit vom erfassten Massenspektrum (39) ein für die Zusammensetzung des Abfallgegenstandes (2) repräsentatives Signal ausgebbar ist.

18. Analyseautomat (7) nach Anspruch 17, **gekennzeichnet durch** eine Heizvorrichtung (34), **durch** die die Gasleitung (26) heizbar ist.

19. Analyseautomat (7) nach Anspruch 17 oder 18, **gekennzeichnet durch** ein Regelsystem, **durch** das die Temperatur der Gasleitung (26) auf einen einstellbar vorgebbaren Soll-Wert regelbar ist.

20. Analyseautomat (7) nach einem der Ansprüche 17 bis 19, **gekennzeichnet durch** eine Spüleinrichtung (28), **durch** die Zwischenmessungen ein Reinigungsgas **durch** die Gasleitung (26) leitbar ist.

21. Analyseautomat (8) nach einem der Ansprüche 17 bis 20, **gekennzeichnet durch** eine Positioniereinrichtung (13), **durch** die die Sonde (14) automatisch an einen Abfallgegenstand (2) heranfahrbar ist.

22. Analyseautomat (8) nach einem der Ansprüche 17 bis 21, **dadurch gekennzeichnet, dass** die Verdampfungseinrichtung (24) einen auf den Abfallgegenstand (2) richtbaren Laser umfasst.
